(19) Europäisches Patentamt
European Patent Office
Office européen des brevets

(11) Publication number: **0 023 046**
**B1**

(12) **EUROPEAN PATENT SPECIFICATION**

(45) Date of publication of patent specification: **14.09.83**

(51) Int. Cl.[3]: **A 61 C 19/00**, A 61 B 1/24

(21) Application number: **80104289.6**

(22) Date of filing: **22.07.80**

# (54) Auxiliary instrument for dental treatment.

(30) Priority: **23.07.79 JP 93525/79**
**17.10.79 JP 142720/79**

(43) Date of publication of application:
**28.01.81 Bulletin 81/4**

(45) Publication of the grant of the patent:
**14.09.83 Bulletin 83/37**

(84) Designated Contracting States:
**DE FR GB IT SE**

(56) References cited:
**DE - C - 807 302**
**DE - C - 847 948**
**US - A - 1 569 504**
**US - A - 2 563 507**
**US - A - 3 060 579**

(73) Proprietor: **Muraoka, Hiroshi**
**No. 8-15, Kyobashi 2-chome Chuo-ku**
**Tokyo (JP)**

(73) Proprietor: **Takahashi, Katumi**
**No. 10-3, Odai 1-chome Adachi-ku**
**Tokyo (JP)**

(72) Inventor: **Muraoka, Hiroshi**
**No. 8-15, Kyobashi 2-chome Chuo-ku**
**Tokyo (JP)**

(74) Representative: **Liebau, Gerhard, Dipl.-Ing.**
**Birkenstrasse 39**
**D-8900 Augsburg 22 (DE)**

**EP 0 023 046 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

Auxiliary instrument for dental treatment

This invention relates to an auxiliary instrument for dental treatment adapted for guiding the centric relation or terminal hinge position of the mandible, of which dentistry requires consideration, into the most suitable position.

Three broad and general classifications for terminal hinge position are currently recognized, that is, the most retruded position of the mandible (Ligmental position), occlusion in light contact (Muscular position) and upper most position of the condyle (Upper most position).

The guidance of terminal hinge position (centric relation) has hitherto been effected by the patient himself in the manner of supporting the mandible using forefingers and thumbs as well as holding the head of the mandible using thumbs and moving the position in a suitable direction relative to the maxilla such as forwards and backwards, up and down or the like. However, in cases where the patient must determine for himself the centric relation by using his fingers it has hitherto been observed that there often takes place the irregularity of centric relation in accordance with the degree of skill of the patient and accordingly the reproducibility of centric relation is hindered, thereby rendering the dental treatment per se unstable. In other words, an auxiliary instrument for dental treatment has hitherto not been proposed which is suitably adapted for assisting the practitioner in guiding the centric relation of the mandible into the most suitable position.

It is an object of this invention to provide a novel auxiliary instrument for dental treatment which can facilitate the guidance of the centric relation of the mandible into the most suitable position in the manner of moving the mandible in a suitable direction relative to the maxilla in place of the manner of guiding the centric relation of the mandible with resort to person's fingers exclusively.

Another object of this invention is to provide an auxiliary instrument for dental treatment which comprises composite members to be assembled with ease and thus is adaptable for the differently shaped mandibles.

A further object of this invention is to provide an auxiliary instrument for dental treatment in which a flexible material is placed, in freely removable manner, on the surface of a mandible retaining plate means as well as the inner peripheral surface of a chin retaining means so that said instrument can be placed fast to the mandible, irrespective of its size, and support it positively.

Fig. 1 is a plan view of a first embodiment of the auxiliary instrument for dental treatment according to this invention.

Fig. 2 is a vertical longitudinal sectional view taken on line II—II of Fig. 1.

Fig. 3 is a slant view illustrating its state in use.

Fig. 4 is a plan view of a second embodiment of the auxiliary instrument for dental treatment according to this invention.

Fig. 5 is a vertical longitudinal sectional view taken on line V—V of Fig. 4.

Fig. 6 is a slant view illustrating composite members in disassembled state.

Fig. 7 is a slant view illustrating its state in use.

Figs. 1 through 3 illustrate the first embodiment of the auxiliary instrument according to this invention.

Reference numeral 1 denotes an auxiliary instrument body having a fixed length and comprising a flat body of synthetic resin or the like. This body 1 defines at the front part a forked mandible retaining plate 2, which, in use, abuts the face under the mandible and also defines at the rear part a handle 3 with its end curved downwardly. And, in the vicinity of the fork base end position of said mandible retaining plate 2, located substantially in the center of the body 1 there is provided, slightly bending backward, a chin retaining means 4 having an arched peripheral wall into which the chin may be inserted. On the body 1 a flexible material 5 having a thickness of about 1 mm is placed, in freely removable manner, on the inside surface of the mandible retaining plates opposite to the chin retaining means 4 and on the inside peripheral surface of the chin retaining means 4. The flexible material thus ensures the adaptation of the body to differently shaped mandibles and, unless needed, may be removed for sterilizing purposes. In this context, it goes without saying that the flexible material 5 may be placed not only on the inside surface of the mandible retaining plate 2, opposite to the chin retaining means 4 but also, for instance, on the whole surface of the retaining plate 2.

Next, reference will be made as to how to use the above device.

As shown in Fig. 3, the body 1 is first placed under the mandible of the patient by means of the handle 3, and then the mandible retaining plate 2, is advanced while abutting the face under the mandible such that the chin retaining means 4 may cover the chin. Thus, the mandible as a whole is not only firmly fixed to the body 1 but also is closely held by the action of said flexible material 5 placed on the mandible retaining plate 2, and the chin retaining means 4. Then, the body 1 supporting the mandible as aforesaid, is moved in a suitable direction relative to the maxilla such as forwards and backwards, up and down or the like, thereby guiding the centric portion of mandible into a most suitable position.

In Figs. 4 through 7 there is illustrated the second embodiment of the auxiliary instrument for dental treatment according to this invention. This auxiliary instrument for dental treatment differs from that of the first embodiment in that the composite elements of the former are arranged to be assembled.

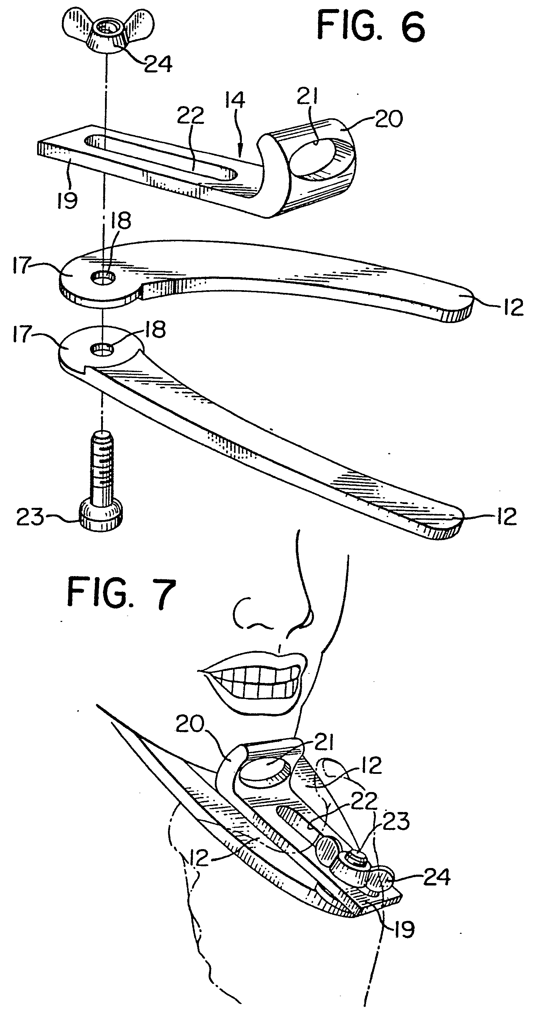

As is clearly seen from Fig. 6, this auxiliary instrument body 11 for dental treatment comprises a forked mandible retaining plate set 12, 12 abutting the face under the mandible, a protruding chin retaining means 14 provided close to the fork base end portion 17 of said mandible retaining plate set 12, 12 and a clamping means 16 for clamping said mandible retaining plate set 12, 12 and that chin retaining means 14. The fork base end portions 17 of the two mandible retaining plates 12 are rounded and their confronting, overlapping faces have a cutaway thickness of about one half of that of each mandible retaining plate 12. Further, said fork base end portion 17 has in its center an opening through which the clamping means is inserted, and the mandible retaining plate is arranged so that it may be opened or closed with the aid of the clamping means 16. The chin retaining means 14 comprises a horizontal base plate 19 and a bend-off substantially L-shaped portion 20 which is defined at the end portion of said base plate in the manner of curving upward gradually, said L-shaped portion 20 having an elliptical opening 21 into which the chin is inserted. A slit 22 extends lengthwise in the horizontal base plate 19, the width of said slit being sufficient for slidable movement of the clamping means 16, whereby said chin retaining means 14 may be shifted forwards and backwards in respect of the base end portion 17 of the mandible retaining plate set 12, 12 towards the front end thereof.

In this context, it is to be noted that the same flexible material being about 1 mm in thickness as used in connection with the first embodiment may be placed, in freely removable manner, on the confronting inside surface of the mandible retaining plate set 12, 12 of the body 11 opposite to the chin retaining plate 14 as well as the inside peripheral surface of the latter. Alternatively, said flexible material, may, be placed on the whole surface of the mandible retaining plate set 12, 12.

Hereinafter, reference will be made as to how to use the above device.

First, the two base end portion 17 of the mandible retaining plate set 12, 12 are aligned to each other a bolt 23 is inserted into the opening 18 defined in the center of said each base end portion 17, this bolt 23 extending through the slit 22 of the horizontal base plate 19, and then a butterfly nut 24 is screwed onto the bolt 23. Assembling of the body 11 is thus completed. Controlling of the butterfly nut 24 makes it possible to open and close the mandible retaining plate set 12, 12 in an optional degree for well fitting differently shaped mandibles and further to position the chin retaining means 14 at a desired front or back position. How to use this assembled instrument is illustrated in Fig. 7. First the rear part of the mandible retaining plate set 12, 12 is grasped by hand and then held so that the front part of the mandible retaining plate set 12, 12 may abut the mandible. The chin may be inserted into the opening 21 of the chin retaining means 14. Thus, the mandible as a whole is firmly fixed to the body 11. Then, the body 11 which has supported the mandible as aforesaid, is moved in a suitable direction relative to the maxilla such as forwards and backwards, up and down or the like, whereby it is rendered possible to guide the centric relation of mandible into a most suitable position.

## Claims

1. An auxiliary instrument for dental treatment adapted for guiding the centric relation or terminal hinge position of the mandible which comprises forked mandible retaining plate means (2, 12) which, in use, abuts the face under the mandible and an upwardly protruding chin retaining means (4, 14) provided in the vicinity of the fork base end portion of said mandible retaining plate means (2, 12) and having a through opening into which, in use, the chin is inserted.

2. An auxiliary instrument for dental treatment as claimed in claim 1 in which said mandible retaining plate means comprises a pair of plate members (12, 12) pivotally joined at their base end portions (17, 17) and arranged to be opened and closed with the aid of a clamping means (23, 24) disposed at the base end portions (17, 17) thereof and said chin retaining means (14) is arranged to be movable forwards and backwards from the fork base end portions (17, 17) of the plate members (12, 12) with the aid of the clamping means 23, 24).

3. An auxiliary instrument for dental treatment as claimed in claim 1 in which a flexible material (5) is placed, in freely removable manner, on the surface of said mandible retaining plate means (2, 12) as well as the inside peripheral surface of said chin retaining means (4, 14).

## Patentansprüche

1. Hilfsinstrument für Zahnbehandlung, welches zur Führung der zentralen Relation oder der Schlußbißstellung des Unterkiefers anwendbar ist, gekennzeichnet durch eine gabelförmige Unterkiefer-Stützplatte (2, 12), die bei Gebrauch an dem Gesicht unter dem Unterkiefer anliegt, und durch ein nach oben vorspringendes Kinnhaltemittel (4, 14), das in der Nähe des Gabelgrundes der Unterkiefer-Stützplatte (2, 12) angeordnet ist und eine Durchgangsöffnung aufweist, in welche bei Gebrauch das Kinn eingeschoben wird.

2. Hilfsinstrument für Zahnbehandlung nach Anspurch 1, dadurch gekennzeichnet, daß die Unterkiefer-Stützplatte ein Paar von Plattenteilen (12, 12) aufweist, die an ihren inneren Grundenden (17, 17) gelenkig miteinander verbunden sind und die mittels einer an ihren Grundenden angeordneten Klemmeinrichtung (23, 24) geöffent und geschlossen werden können, und daß mittels der Klemmeinrichtung (23, 24) das Kinnhaltemittel (14) gegenüber den Grundenden (17, 17) der Plattenteile (12, 12) vorund zurückbeweglich angeordnet ist.

3. Hilfsinstrument für Zahnbehandlung nach Anspruch 1, dadurch gekennzeichnet, daß flexibles Material (5) leicht auswechselbar sowohl auf der Oberfläche der Unterkiefer-Stützplatte (2, 12) als such an der inneren Umfangsfläche des Kinnhaltemittels (4, 14) angeordnet ist.

**Revendications**

1. Instrument auxiliaire pour traitement dentaire, conçu pour guider la partie centrale ou l'extrémité de l'articulation de la mandibule, caractérisé en ce qu'il comprend des plaques fourchues de retenue de mandibule (2, 12) qui, lors de l'utilisation, viennent se placer contre la face située en dessous de la mandibule, ainsi qu'un élément (4, 14) ressortant en saillie vers le haut pour retenir le menton et prévu au voisinage des extrémités de base fourchues des plaques de retenue de mandibule (2, 12), cet élément comportant une ouverture de part en part dans laquelle est introduit le menton lors de l'utilisation.

2. Instrument auxiliaire pour traitement dentaire suivant la revendication 1, caractérisé en ce que les plaques de retenue de mandibule sont au nombre de deux (12, 12) articulées à leurs extrémités de base (17, 17), ces plaques étant conçues pour être ouvertes et formées à l'aide d'un élément de serrage (23, 24) disposé à ces extrémités de base (17, 17), l'élément de retenue du menton (14) étant conçu pour être déplacé vors l'avant et vers l'arrière par rapport aux extrémités de base fourchues (17, 17) des plaques (12, 12) à l'aide de cet élément de serrage (23, 24).

3. Instrument auxiliaire pour traitement dentaire suivant la revendication 1, caractérisé en ce qu'une matière flexible (5) est placée de manière aisément amovible sur la surface des plaques de retenue de mandibule (2, 12), ainsi que sur la surface périphérique intérieure de l'élément de retenue du menton (4, 14).

FIG. 1

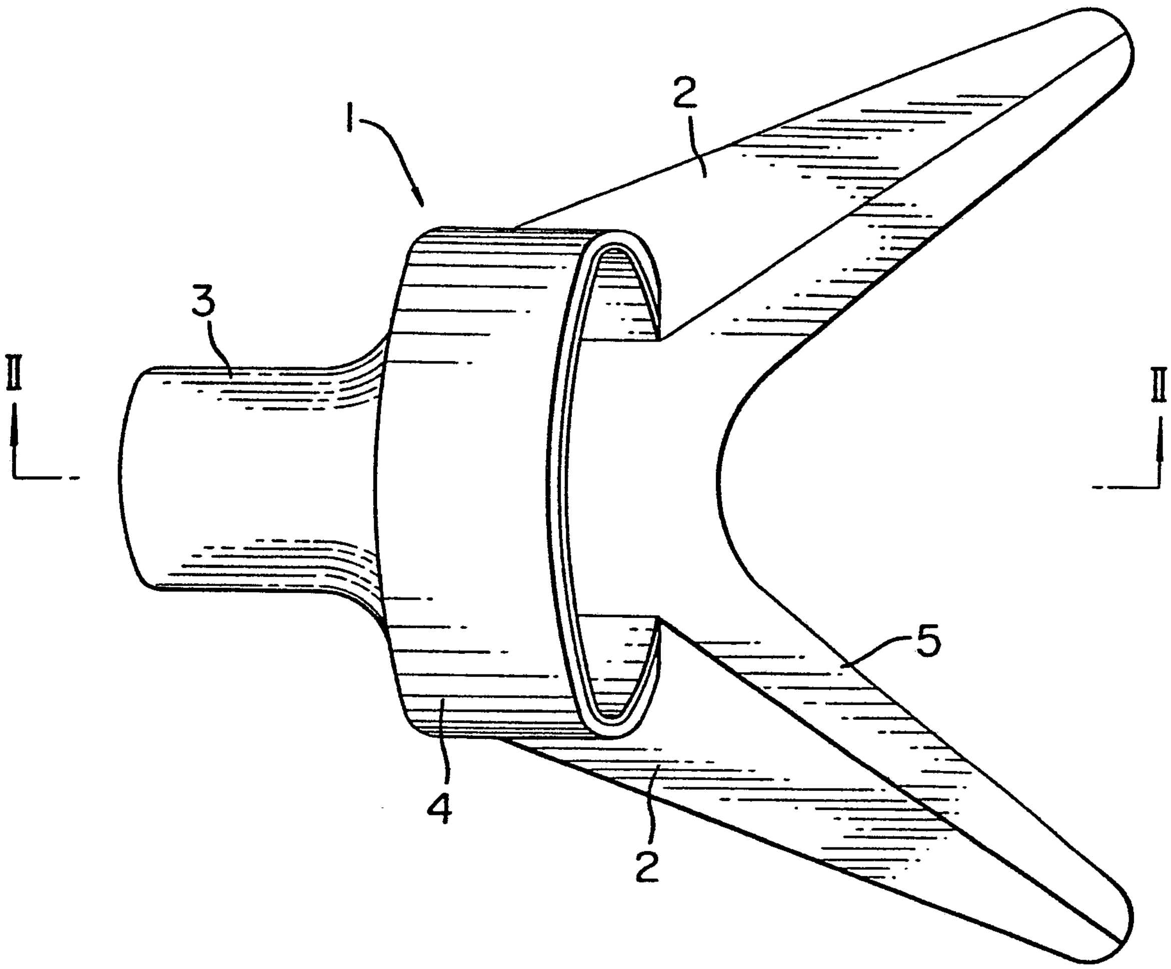


FIG. 2

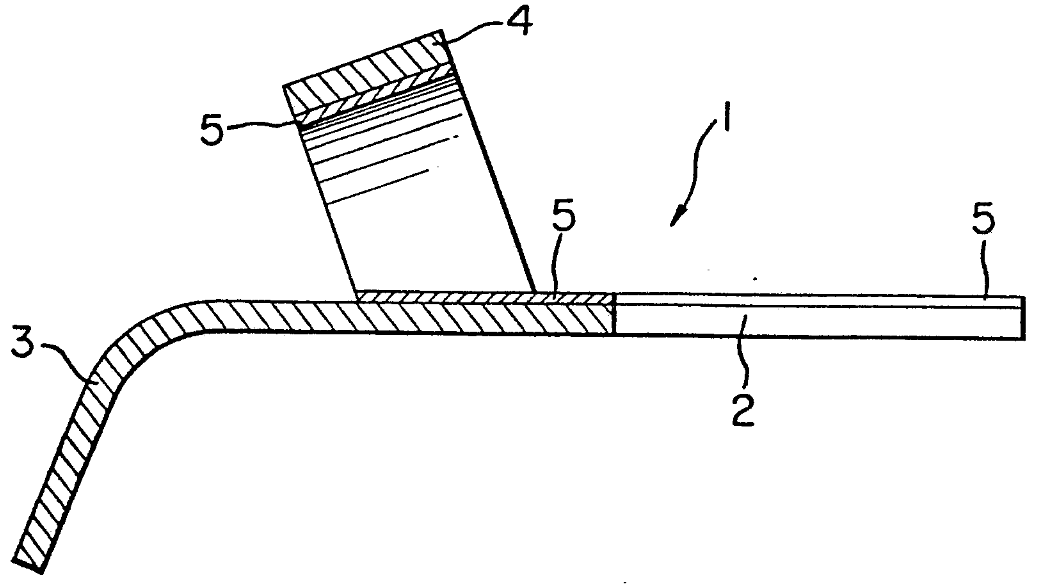

FIG. 3

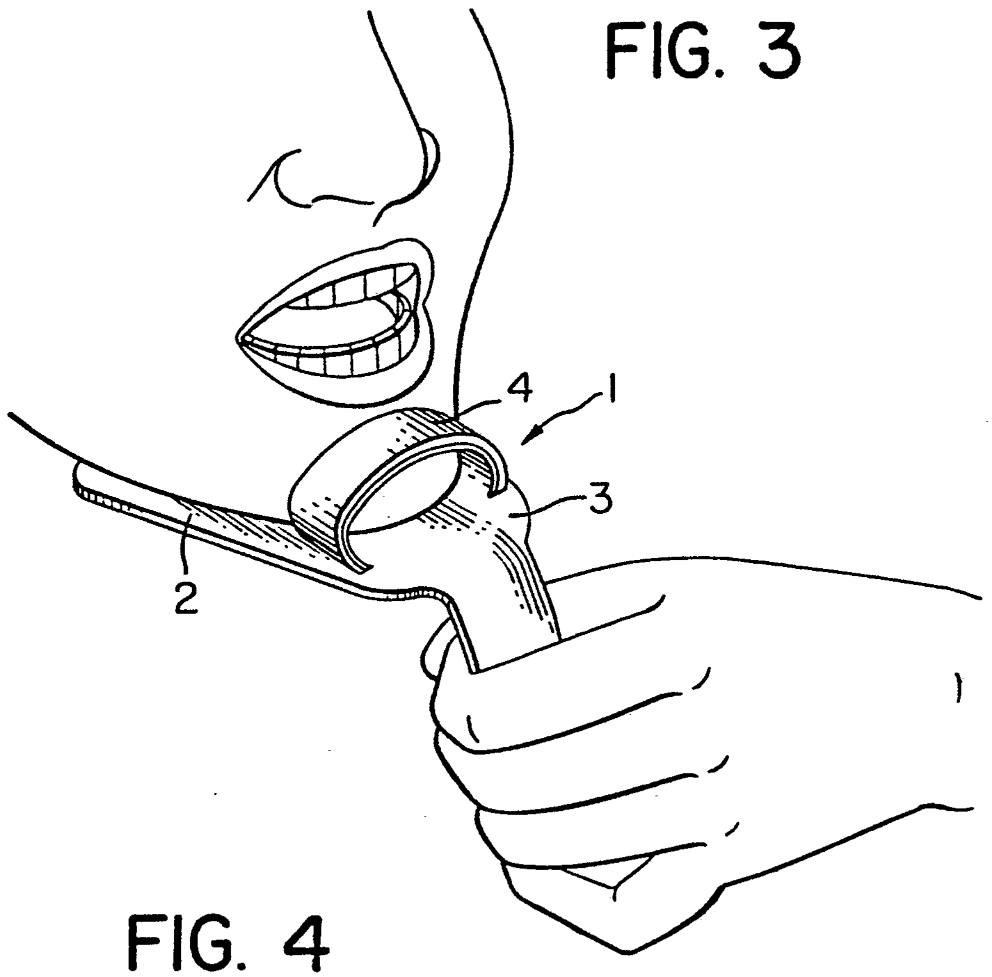


FIG. 4

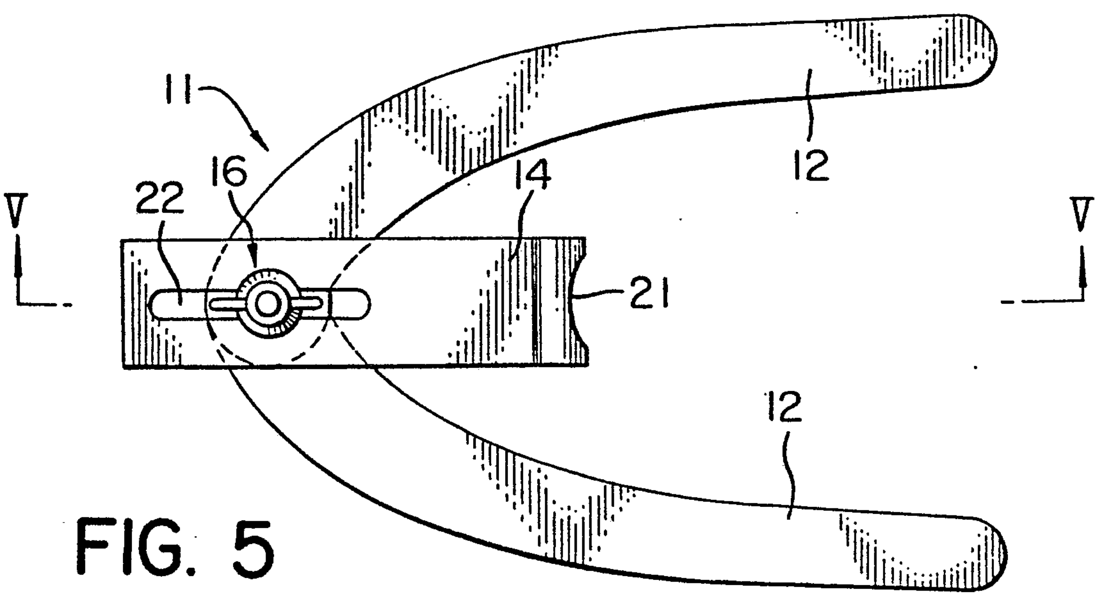


FIG. 5

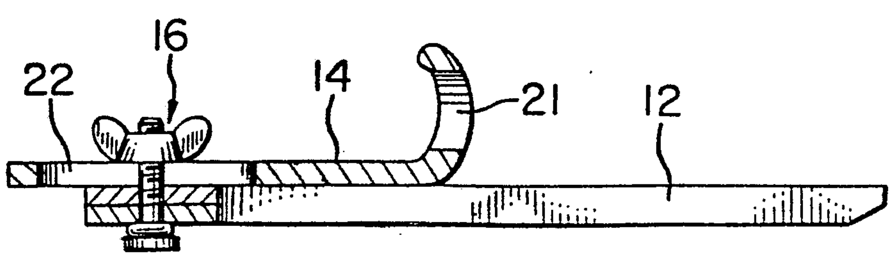

FIG. 6

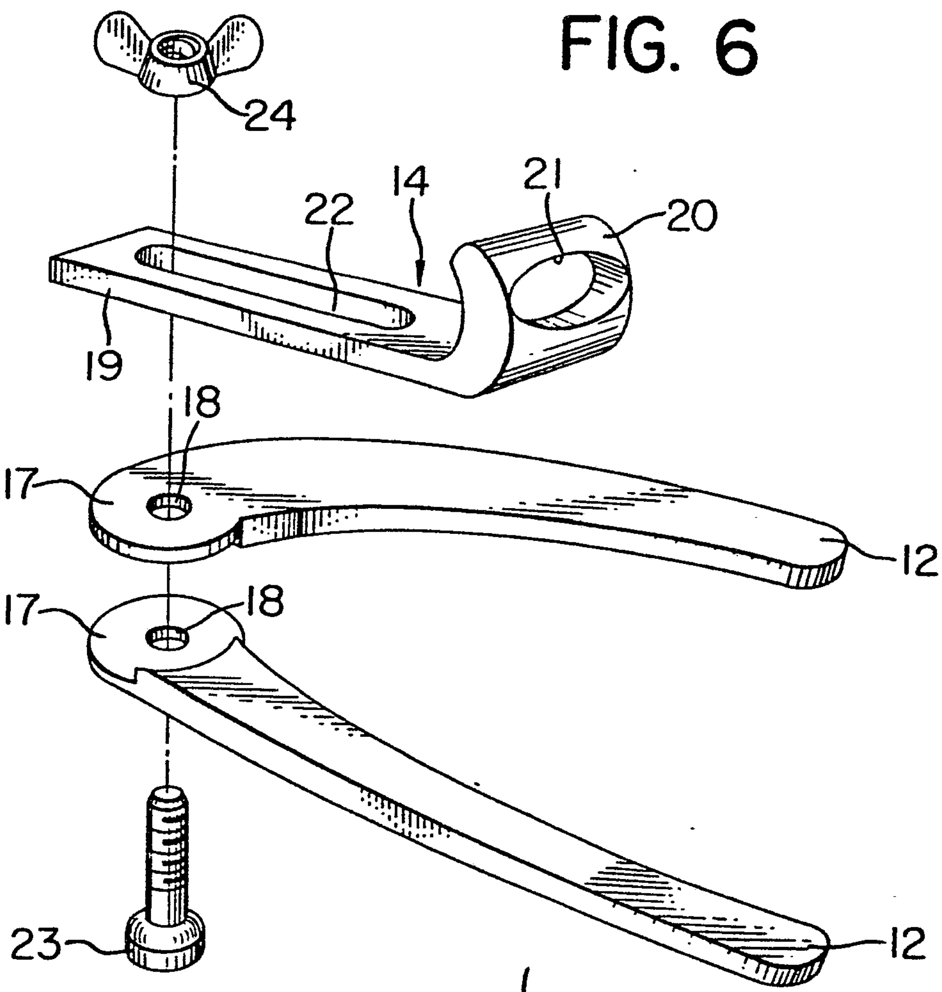


FIG. 7